# EUROPÄISCHE PATENTSCHRIFT

(11) **EP 0 509 587 B1**
(45) Veröffentlichungstag und Bekanntmachung des Hinweises auf die Patenterteilung: **29.11.1995**
(21) Anmeldenummer: 92200988.1
(22) Anmeldetag: 07.04.1992
(51) Int. Cl.: A61B 6/12, A61B 17/22

(54) **Lithotripsie-Arbeitsplatz**
Lithotripsy working place
Poste de travail lithotripsie

(30) Priorität: 13.04.1991 DE 4112148
(43) Veröffentlichungstag der Anmeldung: 21.10.1992
(73) Patentinhaber: Philips Patentverwaltung GmbH, 22335 Hamburg (DE); Philips Electronics N.V., 5621 BA Eindhoven (NL)
(72) Erfinder: Elff, Manfred, Dr., W-2000 Hamburg 65 (DE); Pfeiffer, Wilfried, W-2085 Quickborn (DE); Dirks, Diedrich, W-2000 Hamburg-Norderstedt (DE); Schmidt, Siegfried, Dr., W-2000 Hamburg 53 (DE)
(74) Vertreter: Hartmann, Heinrich, Dipl.-Ing.

(56) Entgegenhaltungen:
- EP-A- 0 348 016
- WO-A-91/07913
- DE-A- 2 909 476
- US-A- 4 930 509

## Beschreibung

Die Erfindung betrifft einen Lithotripsie-Arbeitsplatz mit einer Patientenlagerungsvorrichtung, einer Stoßwellenquelle zur Erzeugung von auf einen raumfesten Punkt (Fokus) fokussierten Stoßwellen, einem Röntgenstrahler, der aus wenigstens zwei bezüglich des Fokus definierten Positionen den Fokus durchstrahlt, und mit einem Bildaufnehmer, der das vom Röntgenstrahler erzeugte Röntgenbild erfaßt.

Ein solcher Lithotripsie-Arbeitsplatz ist aus der EP-OS 286 170 bekannt. Der Röntgenstrahler und der Bildaufnehmer sind dabei an einem U-Arm befestigt, der die Tischplatte der Patientenlagerungsvorrichtung umgreift und um eine zur Längsrichtung der Tischplatte senkrechte horizontale Achse schwenkbar ist. Der U-Arm ist in einem Stativ gelagert. Der Stoßwellenerzeuger ist dabei so angeordnet, daß sich sein Fokus im Schnittpunkt der genannten Achse und des Zentralstrahls befindet.

Die Positionierung eines Patienten in der Weise, daß der zu zertrümmernde (Nieren-) Stein in den Fokus des Stoßwellenerzeugers gelangt, ist bei der bekannten Vorrichtung verhältnismäßig einfach. In einer ersten Position mit senkrecht verlaufendem Zentralstrahl wird der Patient so positioniert, daß der Stein genau auf das markierte Zentrum des Bildaufnehmers - eines Röntgenbildverstärkers - projiziert wird. Der Stein befindet sich dann im Zentralstrahl, kann jedoch oberhalb oder unterhalb des Fokus liegen. In einer zweiten Stellung des U-Arms mit schräg verlaufendem Zentralstrahl wird die Tischplatte daher angehoben oder abgesenkt, bis der Stein wiederum im Zentrum des Bildverstärkers liegt, das durch ein Fadenkreuz oder dergleichen im Röntgenbild sichtbar gemacht werden kann. Danach befindet sich der Stein im Fokus des Stoßwellenerzeugers.

Um die erforderliche Postionierungsgenauigkeit zu erreichen, muß der U-Arm eine hohe mechanische Stabilität aufweisen und ebenso das ihn tragende Stativ. Der U-Arm und das Stativ sind daher teuer und sehr schwer. Darüberhinaus behindert der U-Arm den Zugang zum Patienten auf der Seite der Patientenlagerungsvorrichtung, auf der sich das Stativ befindet.

Aufgabe der vorliegenden Erfindung ist es, einen Arbeitsplatz der eingangs genannten Art so auszugestalten, daß die Positionierung ähnlich leicht und genau ausgeführt werden kann wie bei der bekannten Vorrichtung, aber mit geringeren Anforderungen an die mechanische Präzision. Diese Aufgabe wird erfindungsgemäß durch die im Anspruch 1 angegebenen Maßnahmen gelöst.

Bei der Erfindung hängt die erzielbare Positionierungsgenauigkeit nur davon ab, daß sich die Marke - zumindest in den beiden Positionen, aus denen der Fokus durchstrahlt wird - auf der Verbindungsgeraden zwischen dem Brennfleck des Röntgenstrahlers und dem Fokus der Stoßwellenquelle befindet. Auf die genaue Ausrichtung von Röntgenstrahler und Bildaufnehmer hingegen kommt es für die Positioniergenauigkeit nicht an. Die Halterung für diese Komponenten kann also weniger stabil und daher leichter und preiswerter sein als bei der bekannten Vorrichtung.

Es sei an dieser Stelle erwähnt, daß aus der DE-PS 39 19 083 bereits die Verwendung von im Röntgenbild abbildbaren Marken bei einem Lithotripter bekannt ist. Röntgenstrahler und Bildaufnehmer sind bei dem bekannten Gerät durch die Befestigung an einem C-Bogen aufeinander ausgerichtet. Der C-Bogen ist unabhängig von der Stoßwellenquelle verfahrbar, so daß der Röntgenstrahler keine definierte Position in Bezug auf den Fokus einnimmt.

Um trotzdem eine genaue Positionierung zu erreichen, ist an der Stoßwellenquelle eine Zieleinrichtung befestigt, die (mindestens) vier Marken enthält, von denen je zwei auf Geraden angeordnet sind, die sich im Fokus schneiden. Zur Positionierung des Patienten wird der C-Bogen in eine solche Stellung gebracht, daß sein Zentralstrahl mit einer der beiden erwähnten Geraden zusammenfällt. Da dies in der Regel nicht exakt erreichbar ist, werden die beiden Marken an unterschiedlichen Stellen des Bildaufnehmers bzw. eines damit gekoppelten Monitors abgebildet. Aus der Lage der Marken, die von dem Untersucher z.B. mittels eines Lightpen eingegeben werden muß, kann ein Rechner die Lage des Fokus auf dem Bildschirm errechnen und an der errechneten Stelle eine geeignete elektronische Markierung einblenden. Der Benutzer muß dann den Patienten auf der Patientenlagerungsvorrichtung so verschieben, bis der Stein an derselben Stelle abgebildet wird wie die elektronische Markierung des Fokus. Der gleiche Vorgang muß anschließend für die andere Richtung wiederholt werden.

Dieser Positionierungsvorgang, bei dem auf dem Monitorbild zwei Marken und der elektronisch markierte Fokus erscheinen, ist für den Benutzer sehr verwirrend. Der verfahrbare C-Bogen behindert den Zugang zum Patienten noch mehr als bei dem eingangs erwähnten bekannten Gerät mit einem eine raumfeste Achse schwenkbaren U-Arm.

Weiterhin ist auch aus der US-PS 4,930,509 ein Lithotripsie-Arbeitsplatz bekannt, bei dem zur Unterstützung der Ortung eine im Röntgenbild abbildbare Marke vorgesehen ist. Diese Marke befindet sich auf einer vertikalen Geraden durch den Fokus der am Boden verfahrbaren und in der Höhe verstellbaren Stoßwellenquelle. Wenn der an einem Deckenstativ in x und y-Richtung verfahrbare Deckenstrahler genau über der Marke positioniert wird, ist es möglich, aus der Lage der Marke und des Steins in einem Röntgenfilmbild die x,y-Position des Steins zu bestimmen und den Stein anschließend so zu verschieben, daß auf dem Röntgenfilm sein Bild mit dem Bild der Marke zusammenfällt. Die Position des Steins in z-Richtung läßt sich auf diese Weise nicht bestimmen und in den Fokus des Röntgenstrahlers bringen.

In weiterer Ausgestaltung der Erfindung ist vorgesehen, daß der Bildaufnehmer an einem Deckenstativ befestigt ist. Dadurch ist sichergestellt, daß auch die Halterung für den Bildaufnehmer den Zugang zum Patienten nicht behindert.

Da es für eine genaue Positionierung nicht darauf ankommt, daß Röntgenstrahler und Bildaufnehmer exakt aufeinander ausgerichtet sind, kann der Bildaufnehmer beim Verfahren des Röntgenstrahlers stehenbleiben, wenn seine Eingangsfläche genügend groß ist, um in jeder Position des Röntgenstrahlers das Röntgenstrahlenbild zu erfassen. Man kann nach einer Weiterbildung aber auch mit einem kleineren Bildaufnehmer auskommen durch eine Steuereinrichtung, die Bewegungen von Bildaufnehmer und Röntgenstrahler derart aufeinander abstimmt, daß die Marke - zumindest in den beiden Positionen des Röntgenstrahlers - auf einen kleinen Bereich um das Zentrum der Eingangsfläche des Bildaufnehmers projiziert wird. Es ist möglich, daß der Bildaufnehmer so gekippt wird, daß die Röntgenstrahlung senkrecht auf ihn auftrifft. Es ist aber auch möglich, den Bildaufnehmer bei seiner Bewegung nicht zu kippen, so daß seine optische Achse ihre Richtung nicht verändert.

Die Erfindung wird nachstehend anhand der Zeichnungen näher erläutert. Es zeigen
- Fig. 1: eine bevorzugte Ausführungsform der Erfindung und die
- Fig. 2 und 3: eine schematische Darstellung des erfindungsgemäßen Gerätes in verschiedenen Positionen von Röntgenstrahler und Bildaufnehmer.

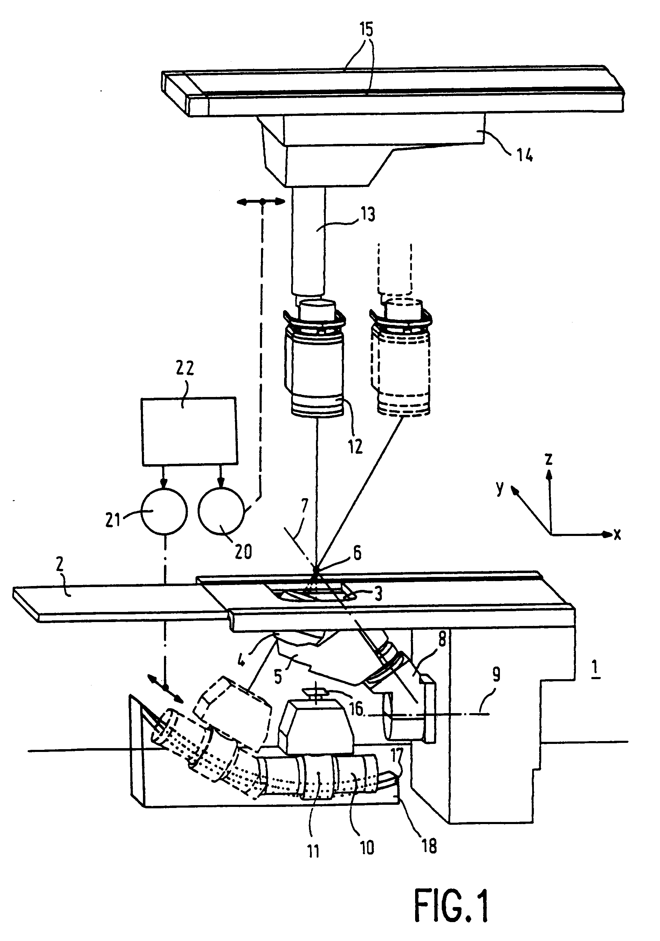

Der in Fig. 1 dargestellte Lithotripsie-Arbeitsplatz umfaßt eine Patientenlagerungsvorrichtung mit einer Tischplatte 2, in der sich eine Öffnung 3 befindet, durch die hindurch Stoßwellen von einer unterhalb der Tischplatte 3 befindlichen Stoßwellenquelle 4 auf den Patienten 24 gerichtet werden können. Die Tischplatte 2 ist auf nicht näher dargestellte Weise in x-, y- und z-Richtung verfahrbar, wobei die x-Richtung mit der Tischlängsrichtung, die y-Richtung mit der dazu senkrechten horizontalen Richtung, die z-Richtung mit der Vertikalen übereinstimmt.

Die Stoßwellenquelle 4 liefert auf einen Punkt 6, den sogenannten Fokus, fokussierte Stoßwellen. Sie ist an einem Schwenkarm 5 befestigt, der um eine schräg durch den Fokus 6 verlaufende Achse 7 schwenkbar ist. Somit kann die Stoßwellenquelle 4 um die Achse 7 geschwenkt werden, ohne daß ihr Fokus 6 seine Lage im Raum ändert. Der Schwenkarm 5 ist seinerseits an einem Lagerhalter 8 befestigt, der um eine unterhalb des Schwenkarms 5 verlaufende Achse 9 schwenkbar ist, die in x-Richtung verläuft. Dadurch kann die Stoßwellenquelle ganz zur Seite geklappt werden.

Insoweit, als bisher beschrieben, ist der Lithotripsie-Arbeitsplatz aus der EP-OS 286 170 bekannt. Hinsichtlich der Vorteile, die dadurch erzielt werden, daß die Stoßwellenquelle um die Achsen 7 und 9 schwenkbar ist, wird auf diese Veröffentlichung verwiesen.

Unterhalb der Tischplatte befindet sich ein Röntgenstrahler 10, von dessen Brennfleck 11 im Betriebszustand ein Strahlenbündel ausgeht, das den Bereich um den Fokus 6 herum auf dem Eingangsschirm eines Röntgenbildverstärkers 12 abbildet. Der Röntgenbildverstärker 12 ist - in z-Richtung verschiebbar - an einem Deckenstativ 13 befestigt, das mittels eines Wagens 14 in den in x-Richtung verlaufenden Schienen 15 verfahrbar ist.

An dem Röntgenstrahler oder dessen Halterung 10 ist eine Marke 16 in einem Abstand vom Brennfleck 11 (z.B. 300 mm) befestigt. Die Marke 16 kann ein Fadenkreuz sein, das auf einer für die Röntgenstrahlung transparenten, zum Zentralstrahl senkrechten Platte durch (Schwer-) Metallstreifen gebildet wird. Die Marke bzw. das Fadenkreuz ist auf der Verbindungsgeraden zwischen dem Brennfleck 11 und dem Fokus 6 angeordnet, so daß die Projektion des Fadenkreuzes auf den Eingangsbildschirm des Röntgenbildverstärkers 12 die Lage des Fokus 6 kennzeichnet.

Der Röntgenstrahler 10 ist auf einer Führungsschiene 17 einer am Boden unterhalb der Tischplatte 2 befestigten Führungseinrichtung 18 verfahrbar. Die Führungsschiene 17 ist um eine in y-Richtung verlaufende Achse durch den Fokus 6 gekrümmt, so daß der Brennfleck 11 des Röntgenstrahlers 10 beim Verfahren eine Kreisbahn um den Fokus 6 beschreibt und die fest mit dem Strahler 10 verbundene Marke 16 immer auf der Verbindungsgeraden zwischen dem Fokus 6 und dem Brennfleck 11 verbleibt.

Mit gestrichelten Linien ist der Strahler 10 in einer Position angedeutet, die sich nach dem Verfahren des Strahlers aus der in ausgezogenen Linien dargestellten Position (in der die Verbindungsgerade zwischen 6 und 11 senkrecht verläuft) in eine Position ergibt, bei der die genannte Verbindungsgerade unter einem Winkel von 30^{o} zur Vertikalen verläuft. Der Bildverstärker 12 muß dabei so nachgeführt werden, daß das vom Röntgenstrahler erzeugte Röntgenbild auf der Eingangsfläche des Bildverstärkers verbleibt; hingegen ist es nicht erforderlich, daß das Fadenkreuz auf dieselbe Stelle des Bildverstärkereingangsschirms projiziert wird. Ein Schwenken des Bildverstärkers, so daß seine Eingangsfläche senkrecht zum Zentralstrahl verläuft, ist auch nicht erforderlich - im Gegensatz zu den bekannten Einrichtungen.

Wie durch strichpunktierte Linien angedeutet, werden der Wagen 14 einerseits und der Röntgenstrahler 10 andererseits mittels je eines Motors 20 bzw. 21 verfahren. Eine Steuereinrichtung 22 sorgt dabei dafür, daß der Bildverstärker 12 und der Röntgenstrahler 10 stets so verschoben werden, daß das Röntgenstrahlenbündel auf die Eingangsfläche des Bildverstärkers 12 trifft.

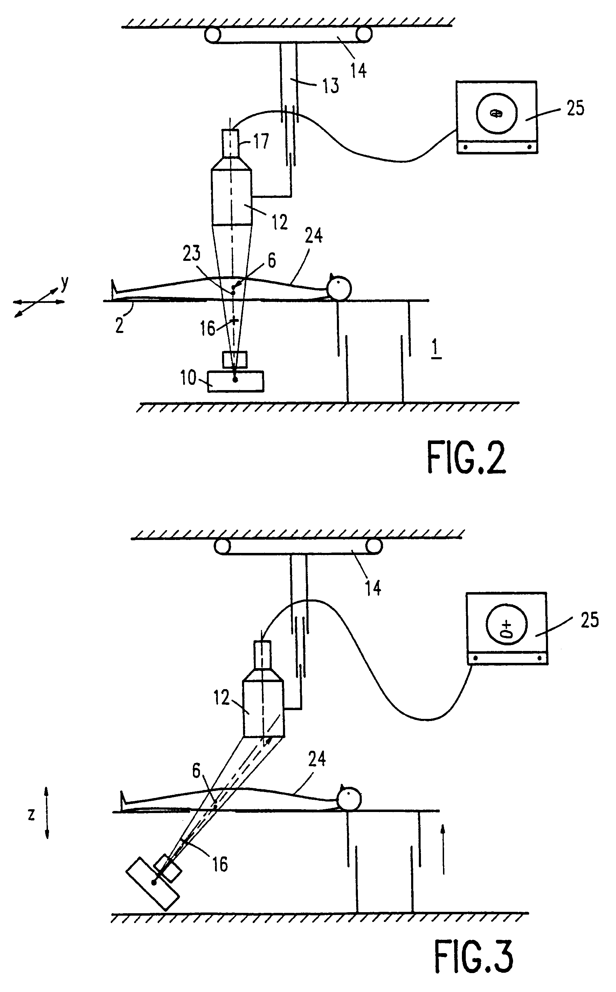

Nachfolgend wird die Positionierung anhand der Fig. 2 und 3 erläutert, in denen die Stoßwellenquelle der Einfachheit halber fortgelassen ist.

Fig. 2 skizziert die Anordnung nach Fig. 1 schematisch bei senkrechtem Strahlengang. Das vom Röntgenstrahler 10 emittierte Röntgenstrahlenbündel erzeugt auf der Eingangsfläche des Bildverstärkers 12 ein Röntgenbild, das das Fadenkreuz 16 beinhaltet sowie einen in dem zu behandelnden Patienten 24 befindlichen (Nieren-) Stein 23, der nach der Positionierung durch den Stoßwellenerzeuger zertrümmert werden soll. Das am Bildverstärkerausgang erzeugte sichtbare Bild wird von einer Fernsehkamera 17 aufgenommen und an einem Monitor 25 wiedergegeben. Der Untersucher verschiebt die Tischplatte in x- und y-Richtung solange, bis sich auf dem Monitor 25 die Bilder der Marke 16 und des Steines 23 decken. Dann befindet sich der Stein auf der vertikalen Verbindungsgeraden zwischen dem Brennfleck des Röntgenstrahlers 10 und dem Fokus 6.

Fig. 3 zeigt die Anordnung nach dem Schwenken des Röntgenstrahlers um ca. 30^{o}. Da sich der Stein 23 unterhalb des Fokus 6 befindet, kommen auf dem Monitorbild der Stein und das Fadenkreuz nicht zur Deckung. Der Benutzer muß dann die Tischplatte nur noch in vertikaler Richtung verfahren (anheben bzw. absenken), bis sich die Bilder von Fadenkreuz und Stein auf dem Monitor decken. Danach befindet sich der Stein im Fokus, und die Behandlung kann beginnen.

Vorstehend wurde die Erfindung anhand einer Einrichtung beschrieben, bei der sich der Röntgenstrahler unterhalb und der Bildverstärker oberhalb der Tischplatte 2 befinden. Die Erfindung ist jedoch auch bei Lithotripsie-Arbeitsplätzen anwendbar, bei den der Röntgenstrahler oberhalb und der Bildverstärker unterhalb der Tischplatte angeordnet sind. - Statt an dem Röntgenstrahler selbst kann die Marke 16 auch an einem fest mit dem Röntgenstrahler verbundenen Teil befestigt sein, z.B. an seiner nicht näher dargestellten Halterung.

## Patentansprüche

1. Lithotripsie-Arbeitsplatz mit einer in den drei Raumrichtungen x, y und z verstellbaren Patientenlagerungsvorrichtung (1), einer Stoßwellenquelle (4) zur Erzeugung von auf einen raumfesten Punkt, dem Fokus (6), fokussierten Stoßwellen, einem Röntgenstrahler (10), der aus wenigstens zwei bezüglich des Fokus definierten Positionen den Fokus (6) durchstrahlt, und mit einem Bildaufnehmer (12), der das vom Röntgenstrahler (10) erzeugte Röntgenbild erfaßt,
dadurch gekennzeichnet, daß an dem Röntgenstrahler oder einem damit fest verbundenen Teil (10) eine im Röntgenbild abbildbare Marke (16) befestigt ist, die sich - zumindest in den beiden Positionen - auf der Verbindungsgeraden zwischen dem Brennfleck (11) des Röntgenstrahlers und dem Fokus (6) befindet, und daß der Röntgenstrahler (10) auf einer Führungsschiene (17) verfahrbar ist, die einen Kreisbogen um den Fokus (6) beschreibt.

2. Lithotripsie-Arbeitsplatz nach Anspruch 1,
dadurch gekennzeichnet, daß der Bildaufnehmer (12) an einem Deckenstativ (13) befestigt ist.

3. Lithotripsie-Arbeitsplatz nach einem der vorhergehenden Ansprüche,
gekennzeichnet durch eine Steuereinrichtung (22), die Bewegungen von Bildaufnehmer und Röntgenstrahler derart aufeinander abstimmt, daß die Marke (16) - zumindest in den beiden Positionen des Röntgenstrahlers - auf einen kleinen Bereich um das Zentrum der Eingangsfläche des Bildaufnehmers (12) projiziert wird.

4. Lithotripsie-Arbeitsplatz nach einem der vorhergehenden Ansprüche,
dadurch gekennzeichnet, daß der Stoßwellenerzeuger unterhalb der Patientenlagerungsvorrichtung (1,2) angeordnet und um eine erste, durch den Fokus (6) und zu den Raumachsen x, y und z schräg verlaufende Achse (7) schwenkbar ist, die mit der Längsrichtung der Patientenlagerungsvorrichtung eine vertikale Ebene bildet.

5. Lithotripsie-Arbeitsplatz nach Anspruch 4,
dadurch gekennzeichnet, daß der Stoßwellenerzeuger (4) von einem Schwenkarm (5) getragen wird, dessen Lager (8) um eine zweite Achse (9) schwenkbar ist, die unterhalb des Lagers in der vertikalen Ebene verläuft.

6. Lithotripsie-Arbeitsplatz nach Anspruch 3,
dadurch gekennzeichnet, daß beim Bewegen des Bildaufnehmers dessen optische Achse ihre Richtung nicht ändert.

## Claims

1. A lithotripsy workstation, comprising a patient supporting device (1) which is adjustable in the three spatial directions x, y and z, a shockwave source (4) for generating shockwaves focused onto a fixed point in space, the focus (6), a X-ray source (10) which irradiates the focus (6) from at least two positions defined relative to the focus, and a image pick-up device (12) which picks up the X-ray image produced by the X-ray source (10), characterized in that a marker (16) which can be displayed in the X-ray image is connected to the X-ray source or a member (10) rigidly connected thereto, which marker is situated, at least in the said two positions, on the connecting line between the focal spot (11) of the X-ray source and the focus (6), and that the X-ray source (10) is displaceable on a guide rail (17) which describes a circular are about the focus (6).

2. A lithotripsy workstation as claimed in Claim 1, characterized in that the image pick-up device (12) is connected to a ceiling stand (13).

3. A lithotripsy workstation as claimed one of the preceding Claims, characterized in that it comprises a control device (22) which matches the movements of the image pick-up device and the X-ray source so that the marker (16) is projected, at least in the said two positions of the X-ray source, onto a small area around the centre of the entrance face of the image pick-up device (12).

4. A lithotripsy workstation as claimed in any one of the preceding Claims, characterized in that the shockwave generator is arranged underneath the patient supporting device (1, 2) and is pivotable about a first axis (7) which extends through the focus (6) and obliquely relative to the spatial axes x, y, z, and which forms a vertical plane in conjunction with the longitudinal direction of the patient supporting device.

5. A lithotripsy workstation as claimed in Claim 4, characterized in that the shockwave generator (4) is supported by a pivot arm (5) whose support (8) is pivotable about a second axis (9) which extends in the vertical plane underneath the support.

6. A lithotripsy workstation as claimed in Claim 3, characterized in that the direction of the optical axis of the image pick-up device does not change during the movement of the image pick-up device.

## Revendications

1. Poste de travail de lithotripsie comprenant un dispositif de positionnement du patient (1) déplaçable dans les trois directions dans l'espace x, y et z, une source d'ondes de choc (4) destinée à générer des ondes de choc focalisées sur un point fixe dans l'espace, le foyer (6), un émetteur de rayonnement X (10), qui rayonne à travers le foyer (6), depuis au moins deux positions définies par rapport au foyer, et comprenant un capteur d'image (12), qui capte l'image de rayons X produite par l'émetteur de rayonnement X (10), caractérisé en ce qu'une marque (16) visualisable dans l'image de rayons X est fixée à l'émetteur de rayonnement X ou à une pipe reliée rigidement à ce dernier (10), laquelle marque se trouve, du moins dans les deux positions, sur la ligne droite reliant le foyer (11) de l'émetteur de rayonnement X et le foyer (6), et que l'émetteur de rayonnement X (10) peut être déplacé sur une glissière de guidage (17), qui décrit un arc de cercle autour du foyer (6).

2. Poste de travail de lithotripsie suivant la revendication 1, caractérisé en ce que le capteur d'image (12) est fixé à un statif plafonnier (13).

3. Poste de travail de lithotripsie suivant l'une quelconque des revendications précédentes, caractérisé par un dispositif de commande (22), qui accorde les mouvements du capteur d'image et de l'émetteur de rayonnement X l'un à l'autre, de façon telle que la marque (16), du moins dans les deux positions de l'émetteur de rayonnement X, soit projetée sur une petite zone autour du centre de la surface d'entrée du capteur d'image (12).

4. Poste de travail de lithotripsie suivant l'une quelconque des revendications précédentes, caractérisé en ce que la source d'ondes de choc est disposée en dessous du dispositif de positionnement du patient (1, 2) et peut pivoter autour d'un premier axe (7) s'étendant à travers le foyer (6) et obliquement par rapport aux axes x, y et z, lequel axe (7) forme un plan vertical avec la direction longitudinale du dispositif de positionnement du patient.

5. Poste de travail de lithotripsie suivant la revendication 4, caractérisé en ce que la source d'ondes de choc (4) est supportée par un bras pivotant (5), dont le palier (8) peut pivoter autour d'un deuxième axe (9), qui s'étend sous le palier dans le plan vertical.

6. Poste de travail de lithotripsie suivant la revendication 3, caractérisé en ce que lors du déplacement du capteur d'image, son axe optique ne change pas d'orientation.
